# EUROPEAN PATENT APPLICATION

(11) **EP 1 086 697 A2**
(43) Date of publication of application: **28.03.2001**
(21) Application number: 00128041.1
(22) Date of filing: 07.12.1992
(51) Int. Cl.: A61K 31/57

(54) **New combination of formoterol and budesonide**

(30) Priority: 18.12.1991 EP 91311761
(62) Divisional of application: 92924683.3
(71) Applicant: AstraZeneca AB, 151 85 Södertälje (SE)
(72) Inventor: Carling, Christer, c/oAstra Zeneca R & D Lund, 22187 Lund (SE); Trofast, Jan, c/oAstra Zeneca R & D Lund, 22187 Lund (SE)

(57) **Abstract**

The invention relates to a novel combination of drugs useful for the treatment of respiratory disorders.

## Description

### Field of the invention

This invention relates to improvements in the treatment of mild as well as severe asthma and other respiratory disorders, More particularly, it relates to the use of a bronchodilator in combination with a steroidal antiinflammatory drug for the treatment of respiratory disorders such as asthma, and to pharmaceutical compositions containing the two active ingredients. It emphasizes the use of a long-acting bronchodilator which provides rapid relief of symptoms.

### Background of the invention

There have recently been significant advances in our understanding of asthma. Despite many advances, both in awareness of the disease by doctors and patients alike, coupled with the introdction of very powerful and effective anti-asthma drugs, asthma remains a poorly understood and often poorly treated disease. Previously, contraction of airway smooth muscles has been regarded as the most important feature of asthma. Recently there has been a marked change in the way asthma is managed, stemming from the fact that asthma is recognized as a chronic inflammatory disease. Uncontrolled airway inflammation may lead to mucosal damage and structural changes giving irrversible narrowing of the airways and fibrosis of the lung tissue. Therapy should therefore be aimed at controlling symptoms so that normal life is possible and at the same time provide basis for treating the underlying inflammation.

The most common cause for poor control of asthma is poor compliance with the long-term management of chronic asthma, particularly with prophylatic treatments, such as inhaled steroids, which do not give immediate symptom relief. Patients will readily take β₂-agonist inhalers, since these provide rapid relief of symptoms, but often do not take prophylactic therapy, such as inhaled steroids, regularly because there is no immediate symptomatic benefit. They also counteract down regulation of β₂-adrenoceptor agonists.

Formoterol, (N-[2-hydroxy-5-[1-hydroxy-2- [[2-(4-methoxyphenyl)-1-methylethyl]amino]ethyl]phenyl] formamide), is an adrenoceptor agonist which selectively stimulates β₂-receptors, thus producing relaxation of bronchial smooth muscle, inhibition of the release of endogenous spasmogens, inhibition of oedema caused by endogenous mediators, and increased mucociliary clearance. Inhaled formoterol fumarate acts rapidly, usually within minutes which gives the patient immediate confirmation that he has taken an adequat dose and thereby avoiding overdosing of both β-agonist and steroid. Inhaled formoterol also exerts a prolonged bronchodilation, which in clinical trials has been demonstrated as up to 12 hours.

Budesonide, (16,17-butylidenebis(oxy)-11,21-dibydroxygregna-1,4-diene-3,20-diona), may be given in a high inhaled dose (up to 2 mg daily) with very low systemic effects, possibly because of its rapid metabolism. The high rapid systemic elimination of budesonide is due to extensive and rapid hepatic metabolism. Long term clinical studies have shown that inhaled budesonide is a pharmacologically safe drug. High doses of inhaled budesonide are highly effective and well tolerated when used in oral steroid replacement therapy. Budesonide represents a logical safe and effective therapy for long term control of asthma.

The inhaled route of administration enables the dose to be delivered directly to the airways. By this type of administration, it is possible to give a small dose and thereby minimizing unwanted side-effects. The drawbacks of the currently available bronchodilators are their relatively short duration of action. By using a compound with long duration e.g. formoterol it would be possible to avoid the nocturnal asthma, which so often causes considerable anxiety and debility to the patients. Formoterol gives less nocturnal waking than the commonly used short-acting agonists like salbutamol, terbutaline and the like. Formoterol has been registered for oral administration in Japan since 1986.

Pharmaceutical combinations of long-acting β₂-agonists and steroids are disclosed in two European applications, EP 416950 which discloses the combination of salmeterol and beclomethasone, and EP 416951 which discloses the combination of salmeterol and fluticasone propionate.

In Ann. Allergy 1989, 63 (3), p. 220-224 the use of a β₂-agonist, i.e. formoterol and a steroid, i.e, budesonide seperately are mentioned. It is not disclosed a pharmaceutical combination including both formoterol and budesonide, or the use of the two compounds in combination therapy. The use of a β₂-agonist and a steroid separately is also mentoined in Lung (1990), 168, no. supp, p. 105-110.

### Outline of the Invention

The present invention is based on the concept of a novel combination therapy whereby formoterol (and/or a physiologically acceptable salt and/or solvate thereof) and budesonide are administrated simultaneously, sequentially or seperately by inhalation. This combination has not only a greater efficiency and duration of bronchodilator action but the combination also has a rapid onset of action. This new feature is of utmost importance in order to establish a higher compliance for patients and it provides a rescue medicine thereby avoiding the necessity for the patient of carrying two different inhalers. This simplifies life for patients considerably and makes life more comfortable and secure. The rapid onset of the long-acting β₂-agonist gives the patient immediate confirmation that he has taken an adequate dose and thereby avoiding overdosing of both β₂-agonist and steroid. Since the use of formoterol instead of salmoterol gives a much more rapid onset the combinations according to the invention have a number of advantages compared to the combinations disclosed i EP 416950 and EP 41651. The combination according to present invention permits a twice daily dosing regime as a basic treatment of asthma, particularly nocturnal asthma,

The present invention provides a medicament containing, separately, or together, (i) formoterol (and/or a physiologically acceptable salt and/or solvate thereof) and (ii) budesonide for simultaneous, sequential or separate administration by inhalation in the treatment of respiratory disorder.

The invention also provides a pharmaceutical composition for administration by inhalation in the treatment of respiratory disorder which composition comprises formoterol (and/or a physiologically acceptable salt and/or solvate thereof) and budesonide.

According to another aspect of the invention there are provided pharmaceutical compositions comprising effective amounts of formoterol (and/or a physiologically acceptable salt and/or solvate thereof) and budesonide as a combined preperation for simultaneous, sequential or seperate administration by inhalation in the treatment of respiratory disorder.

The invention further provides formoterol (and/or a physiologically acceptable salt and/or solvate thereof) and budesonide for use in combination therapy by simultaneous, sequential or seperate administration by inhalation in the treatment of respiratory disorder.

Further the invention provides the use of formoterol (and/or a physiologically acceptable salt and/or solvate thereof) in the manufacture of a medicament for combination therapy where formoterol (and/or a physiologically acceptable salt and/or solvate thereof) and budesonide are administered simultaneously, sequentially or seperately by inhalation in the treatment of respiratory disorder and the use of budesonide in the manufacture of a medicament for combination therapy where formoterol (and/or a physiologically acceptable salt and/or solvate thereof) and budesonide are administered simultaneously, sequentially or separately by inhalation in the treatment of respiratory disorder.

The invention additionally relates to the use of formoterol (and/or a physiologically acceptable salt and/or solvate thereof) and budesonide in the manufacture of a medicament for combination therapy for simultaneous, sequential or seperate administration of formoterol and budesonide by inhalation in the treatment of respiratory disorder.

According to a further feature of the invention there is provided a method of treating respiratory disorder which comprises the simultaneous, sequential or separate administration by inhalation of effective amounts of formoterol (and/or a physiologically acceptable salt and/or solvate thereof) and budesonide.

Suitable physiologically salts of formoterol include acid addition salts derived from inorganic and organic acids, such as the hydrochloride, hydrobromide, sulphate, phosphate, maleate, fumarate, tartrate, citrate, benzoate, 4-methoxybenzoate, 2- or 4-hydroxybenzoate. 4-chlorobenzoate, p-coluenesulphonate, methanesulphonate, ascorbate, salicylate, acetate succinate, lactate, glutarate, gluconate, tricarballylate, hydroxynaphthalenecarboxylate or oleate. Formoterol is preferably used in the form of its fumarate salt and as a dihydrate

The ratio of formoterol to budesonide used according to the invention is preferably within the range of 1:4 to 1:70. The two drugs may be administered separately in the same ratio.

The intended dose regimen is a twice daily administration, where the suitable daily dose of farmoterol is in the range of 6 to 100 µg with a preferred dose of 6-48 µg and the suitable daily dose for budesonide is 50 to 4800 µg with a preferred dose of 100-1600 µg. The particular dose used will strongly depend on the patient (age, weight etc) and the severity of the disease (mild, moderate, severe asthma etc).

For administration, the combination is suitably inhaled from a nebulizer, from a pressurized metered dose inhaler or as a dry powder from a dry powder inhaler (e.g. as sold under the trade mark Turbuhaler) or from a dry powder inhaler utilizing gelatine, plastic or other capsules, cartridges or blister packs.

A diluent or carrier, generally non-toxic and chemically inert to the medicament e.g. lactose, dextran, mannitol or glucose or any additives that will give the medicament a desired taste, can be added to the powdered medicament.

Examples of the preparation of suitable dosage forms according to the invention include the following: Formoterol fumarate dihydrate and budesonide (optionally premicronized) are mixed in the proportions given above. The agglomerated, free-flowing micronized mixture may be filled into dry powder inhaler such as sold under the trade mark Turbuhaler. When a capsule system issued, it is desirable to include a filler in the mixture.

The micronized mixture may be suspended or dissolved in a liquid propellant mixture which is kept in a container that is sealed with a metering valve and fitted into a plastic actuator. The propellants used may be chlorofluorocarbons of different chemical formulae. The most frequently used chlorofluorocarbon propellants are trichloromonofluoromethane (propellant 11), dichlorodifluoromethane (propellant 12), dichlorotetrafluoroethane (propellant 114), tetrafluoroethane (propellant 134a) and 1,1-difuoroethane (propellant 152a). Low concentrations of a surfactant such as sorbitan trioleate, lecithin, disodium dioctylsulphosuccinate or oleic acid may also be used to improve the physical stability.

The invention is further illustrated by way of example with reference to the following Examples.

### Example 1 - Dry Powder Inhaler (Turbuhaler)

| Active ingredient | Per dose |
|---|---|
| Formoterol (as fumarate dihydrate) | 12 µg |
| Budesonide | 200 µg |

The storage unit of the inhaler is filled with sufficient for at least 200 doses.

| Active ingredient | Per dose |
|---|---|
| Formoterol (as fumarate dihydrate) | 24 µg |
| Budesonide | 200 µg |
| The storage unit is filled with sufficient for at least 200 doses. | |

| Active ingredient | Per dose |
|---|---|
| Formoterol (as fumarate dihydrate) | 12 µg |
| Budesonide | 100 µg |
| The storage unit is filled with sufficient for at least 200 doses. | |

### Example 2 - Metered dose inhaler

| Active ingredient | Per dose |
|---|---|
| Formoterol (as fumarate dihydrate) | 12 µg |
| Budesonide | 200 µg |
| Stabilizer | 0.1 - 0.7 mg |
| Propellant | 25 - 100 µl |

| Active ingredient | Per dose |
|---|---|
| Formoterol (as fumarate dillydrate) | 24 µg |
| Budesonide | 200 µg |
| stabilizer | 0.1 - 0.7 mg |
| Propellant | 25 - 100 µl |

| Active ingredient | Per dose |
|---|---|
| Formoterol (as fumarate dihydrate) | 12 µg |
| Budesonide | 200 µg |
| Stabilizer | 0.1 - 0.7 mg |
| Propellant | 25 - 100 µl |

### Example 3 - Metered dose dry powder formulation

| Active ingredient | Per dose |
|---|---|
| Formoterol (as fumarate dihydrate) | 12 µg |
| Budesonide | 200 µg |
| Lactose | up to 5, 12.5 or 25 mg |

| Active ingredient | Per dose |
|---|---|
| Formoterol (as fumarate dihydrate) | 24 µg |
| Budesonide | 200 µg |
| Lactose | up to 5, 12.5 or 25 mg |

| Active ingredient | Per dose |
|---|---|
| Formoterol (as fumarate dihydrate) | 12 µg |
| Budesonide . | 100 µg |
| Lactose | up to 5, 12.5 or 25 mg |

## Claims

1. A medical product comprising, together,
(i) formoterol or a physiologically acceptable salt thereof, or a solvate of said salt, or a solvate of formoterol; and
(ii) budesonide as a combined preparation for administration by inhalation.

2. A medical product according to claim 1, which comprises, as component (i), formoterol fumarate dihydrate.

3. A medical product according to claim 1 or 2, which comprises a non-toxic diluent or carrier.

4. A medical product according to claim 3, in which the non-toxic diluent or carrier is lactose.

5. A medical product according to any of the preceding claims, in which components (i) and (ii) are in dry powder form.

6. A medical product according to any of the preceding claims, in which the ratio of the component (i) to component (ii) is in the range 1:4 to 1:70.

7. A medical product according to any of the preceding claims, which contains component (i) and (ii) in unit dose form.

8. A medical product according to any of the preceding claims in which components (i) and (ii) are for administration by inhalation in the treatment of a respiratory disorder.

9. A medical product according to any of claims 1 to 8 in which components (i) and (ii) are for administration by inhalation in the treatment of asthma.

10. A medical product according to any of the preceding claims, in which the administration is by inhalation from a dry powder inhaler.

11. A medical product according to any of claims 1 to 9 in which the administration is by inhalation from a pressurized metered dose inhaler.

12. A medical product according to any of claims 1 to 9 in which the administration is by inhalation from a nebulizer.

13. A pharmaceutical composition for administration by inhalation, which comprises
(i) formoterol or a physiologically acceptable salt thereof, or a solvate of the salt, or a solvate of formoterol; and
(ii) budesonide.

14. A composition according to claim 13 which comprises, as component (i), formoterol fumarate dihydrate.

15. A composition according to claim 13 or 14 which comprises a non-toxic diluent or carrier.

16. A composition according to claim 15 in which the non-toxic diluent or carrier is lactose.

17. A composition according to any of claims 13 to 16 in which the ratio of the component (i) to component (ii) is in the range 1:4 to 1:70.

18. A composition according to any of claims 13 to 17 in unit dose form.

19. A composition according to any of claims 13 to 18 in which components (i) and (ii) are each in micronised form.

20. A composition according to claim 19 which is in agglomerated form.

21. A dry powder inhaler which contains a composition as claimed in claim 20.

22. A capsule, cartridge or blister pack for a dry powder inhaler, the capsule, cartridge or blister pack containing a composition as claimed in claim 20.

23. A composition as claimed in any one of claims 13 to 20 for use in the treatment by inhalation of a respiratory disorder.

24. A composition as claimed in any one of claims 13 to 20 for use in the treatment by inhalation of asthma.

25. A composition according to any one of claims 13 to 20 for use in the treatment of nocturnal asthma.

26. A composition according to any one of claims 13 to 20 suitable for twice daily dosing.

27. Use of
(i) formoterol, or a physiologically acceptable salt thereof, or a solvate of said salt, or a solvate of formoterol; and
(ii) budenoside,
in the manufacture of a combined preparation for administration by inhalation in the treatment of respiratory disorder.

28. Use according to claim 27, in which component (i) is formoterol fumarate dihydrate.

29. Use according to claim 27 or 28 in which the ratio of the component (i) to component (ii) is in the range 1:4 to 1:70.

30. Use according to any of claims 27 to 29 in which the dose regime is twice daily and the dose of component (i) is 6 - 100 µg per day and the dose of component (ii) is 50 - 4800 µg per day.

31. Use according to claim 30 in which the dose of component (i) is 6 - 48 µg per day and the dose of component (ii) is 100 to 1600 µg per day.

32. Use of formoterol, or a physiologically acceptable salt thereof, or a solvate of said salt, or a solvate of formoterol, in the manufacture of a medical product as defined in claim 1, for administration by inhalation in the treatment of nocturnal asthma.

33. Use of budenoside in the manufacture of a medical product as defined in claim 1, for administration by inhalation in the treatment of nocturnal asthma.
